# EUROPEAN PATENT APPLICATION

(11) **EP 2 490 098 A1**
(43) Date of publication of application: **22.08.2012**
(21) Application number: 12152798.0
(22) Date of filing: 27.01.2012
(51) Int. Cl.: G06F 1/20

(54) **Natural exhaustion type ultrasonic diagnostic apparatus**

(30) Priority: 18.02.2011 KR 20110014743; 18.02.2011 KR 20110014744
(71) Applicant: Samsung Medison Co., Ltd., Nam-myun Hongchun-gun Kangwon do 250-875 (KR)
(72) Inventor: Woo, Kyeong Gu, Gyeonggi-do (KR); KANG, Hak Il, Seoul (KR); Ko, Jae Yong, Seoul (KR)
(74) Representative: Schmid, Wolfgang

(57) **Abstract**

A natural exhaustion type ultrasonic diagnostic apparatus. The natural exhaustion type ultrasonic diagnostic apparatus includes a case unit (30) forming the outer portion of a control panel for ultrasonic diagnostic apparatuses, a suction part (36) provided at the lower portion of the case unit and forming a path through which air is sucked into the case unit, and an exhaust part (38) provided at the upper portion of the case unit and forming a path through which the air introduced into the case unit through the suction part is discharged to the outside.

## Description

### BACKGROUND

### 1. Field

Embodiments of the present invention relate to a natural exhaustion type ultrasonic diagnostic apparatus which exhausts heated air without a separate power device to reduce noise and power consumption.

### 2. Description of the Related Art

An ultrasonic diagnostic apparatus irradiates an ultrasonic signal from the skin of the body of a subject toward a desired portion within the body, and obtains an image regarding a tomogram of soft tissue or a bloodstream using the reflected ultrasonic signal (ultrasonic echo signal).

The ultrasonic diagnostic apparatus has a small size, is inexpensive, displays an image in real time, and does not cause X-ray exposure, as compared to an X-ray diagnostic apparatus, a computerized tomography (CT) scanner, a magnetic resonance imager (MRI) and a nuclear medicine diagnostic apparatus, thus having high safety. Therefore, the ultrasonic diagnostic apparatus is widely used for diagnoses of the heart and the abdomen, and urinary and maternity diagnoses.

The ultrasonic diagnostic apparatus includes a probe which transmits an ultrasonic signal to a subject and receives the ultrasonic signal reflected by the subject in order to obtain an ultrasonic image of the subject.

The probe is connected to a control panel, and thus an operating signal is transmitted from the control panel to the probe and an image measured by the probe is transmitted to the control panel.

Since a heat source member including a hard disk drive and a central processing unit is installed within the control panel, a separate cooling device to cool the heat source member is installed.

The above-described technical configuration is only a background to assist in understanding embodiments of the present invention, and does not mean the conventional technique is well known in the art.

In order to dissipate heat from the inside of the control panel, a cooling fan and a motor to rotate the fan are installed, and thus operating noise and power consumption are increased. Therefore, measures to improve these negative aspects are required.

### SUMMARY

Therefore, it is an aspect of the present invention to provide a natural exhaustion type ultrasonic diagnostic apparatus which exhausts heated air without a separate power device to reduce noise and power consumption.

Additional aspects of the invention will be set forth in part in the description which follows and, in part, will be obvious from the description, or may be learned by practice of the invention.

In accordance with one aspect of the present invention, a natural exhaustion type ultrasonic diagnostic apparatus includes a case unit forming the outer portion of a control panel for ultrasonic diagnostic apparatuses, a suction part provided at the lower portion of the case unit and forming a path through which air is sucked into the case unit, and an exhaust part provided at the upper portion of the case unit and forming a path through which the air introduced into the case unit through the suction part is discharged to the outside.

The case unit may include a lower case provided with the suction part, and an upper case connected to the upper portion of the lower case and provided with the suction part.

The case may include an inclined guide part provided with a bottom surface inclined toward the exhaust part.

The case unit may include guide diaphragms to guide a channel of air introduced into the guide unit through the suction part and moved to the exhaust part.

The guide diaphragms may protrude along both sides of the channel of air.

The channel of air formed at the inside of the guide diaphragms may be gradually narrowed toward the exhaust part.

A heat source member may contact a channel of air introduced into the guide unit through the suction part and moved to the exhaust part.

The natural exhaustion type ultrasonic diagnostic apparatus may further include a heat dissipation unit to cool the heat source member by thermal conduction.

The heat dissipation unit may include a heat dissipation body contacting the outer surface of the heat source member, and heat dissipation fins protruding from the heat dissipation body to increase a contact area with air.

Noise generated from the inside of the case unit may be 20 decibels or less.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is an exploded perspective view schematically illustrating a portion of a natural exhaustion type ultrasonic diagnosis device in accordance with one embodiment of the present invention;
FIG. 2 is a perspective view illustrating a connection between a heat source member and a heat dissipation unit in accordance with the embodiment of the present invention;
FIG. 3 is a sectional view schematically illustrating a portion of the natural exhaustion type ultrasonic diagnosis device in a disassembled state in accordance with the embodiment of the present invention;
FIG. 4 is a sectional view schematically illustrating a portion of the natural exhaustion type ultrasonic diagnosis device in an assembled state in accordance with the embodiment of the present invention; and
FIG. 5 is a sectional view schematically illustrating an inclined guide part of a natural exhaustion type ultrasonic diagnosis device in accordance with another embodiment of the present invention.

### DETAILED DESCRIPTION

Reference will now be made in detail to exemplary embodiments of the present invention, examples of which are illustrated in the accompanying drawings, wherein like reference numerals refer to like elements throughout.

Hereinafter, a natural exhaustion type ultrasonic diagnosis device in accordance with one embodiment of the present invention will be described with reference to the accompanying drawings. For convenience of description, a natural exhaustion type ultrasonic diagnosis device used as a hand-carried ultrasound (HCU) system will be exemplarily illustrated. Thicknesses of lines and sizes of components shown in the drawings may be exaggerated for precision and convenience of description. Also, the terms used in the following description are terms defined taking into consideration the functions obtained in the description and may be changed in accordance with the intention of an operator or a usual practice. Therefore, the definitions of these terms should be determined based on the whole content of this specification.

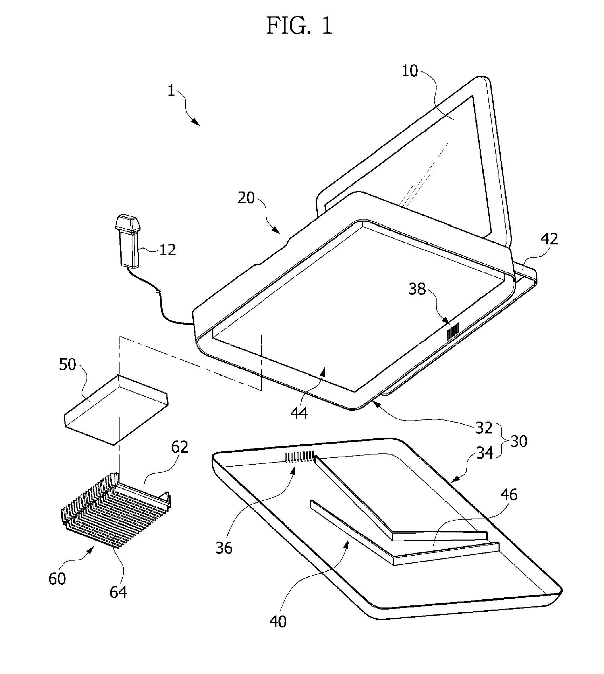

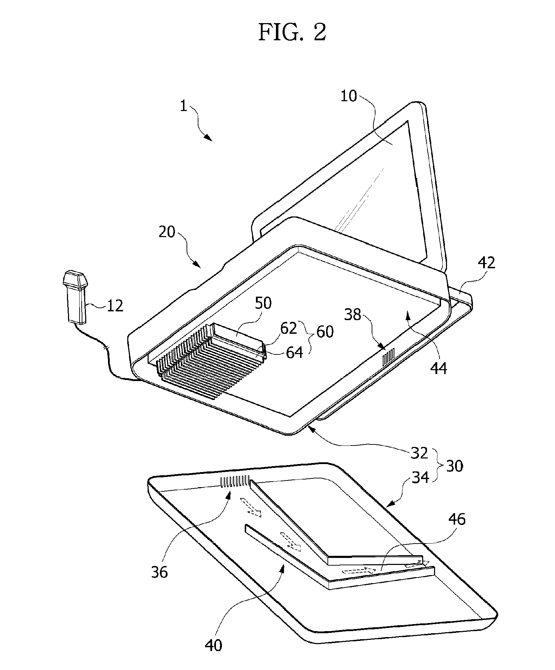

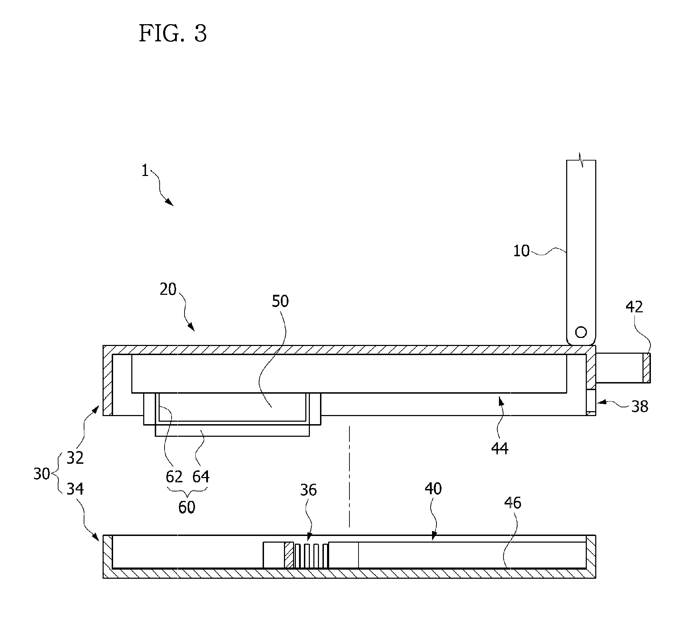

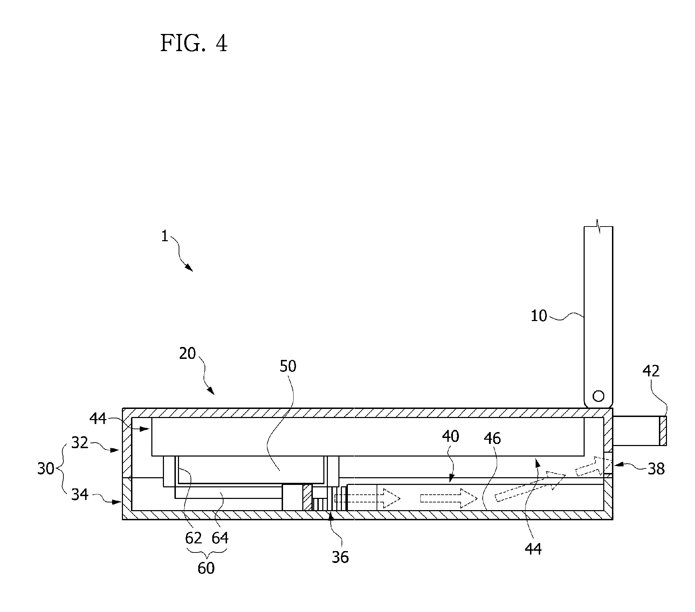

FIG. 1 is an exploded perspective view schematically illustrating a portion of a natural exhaustion type ultrasonic diagnosis device in accordance with one embodiment of the present invention, FIG. 2 is a perspective view illustrating a connection between a heat source member and a heat dissipation unit in accordance with the embodiment of the present invention, FIG. 3 is a sectional view schematically illustrating a portion of the natural exhaustion type ultrasonic diagnosis device in a disassembled state in accordance with the embodiment of the present invention, and FIG. 4 is a sectional view schematically illustrating a portion of the natural exhaustion type ultrasonic diagnosis device in an assembled state in accordance with the embodiment of the present invention.

As shown in FIGS. 1 to 4, a natural exhaustion type ultrasonic diagnostic apparatus 1 in accordance with the embodiment of the present invention includes a case unit 30 forming the outer portion of a control panel 20 for ultrasonic diagnostic apparatuses, a suction part 36 provided at the lower portion of the case unit 30 and forming a path through which air is sucked into the case unit 30, and an exhaust part 38 provided at the upper portion of the case unit 30 and forming a path through which the air introduced into the case unit 30 through the suction part 36 is discharged to the outside.

The natural exhaustion type ultrasonic diagnostic apparatus 1 in accordance with the embodiment of the present invention is used as a hand-carried ultrasound (HCU) system and is easy to carry, and the inside of the natural exhaustion type ultrasonic diagnostic apparatus 1 dissipates heat without separate power.

A probe 12 serving as ultrasonic diagnosis equipment is connected to the control panel 20, and a display 10 to display an ultrasonic image of a subject is installed at the upper portion of the control panel 20 (based on FIG. 1).

The case unit 30 is installed at the outer portion of the control panel 20 for ultrasonic diagnostic apparatuses. The suction part 36 forming the path through which air is sucked into the case unit 30 is provided at the lower portion of the case unit 30, and the exhaust part 38 through which air is discharged to the outside of the case unit 30 is provided at the upper portion of the case unit 30.

A handle 42 is installed at one side of the case unit 30 so as to be gripped by a user, and thus movement of the natural exhaustion type ultrasonic diagnostic apparatus 1 is more conveniently carried out.

Air introduced into the case unit 30 through the suction part 36 is heated while cooling a heat source member 50, and is then discharged to the outside of the case unit 30 through the exhaust part 38 provided at the upper portion of the case unit 30.

The exhaust part 38 is located above the suction part 36 so that air is naturally exhausted using upward movement of air heated while cooling the heat source member 50 due to convection, and the installed positions of the suction part 36 and the exhaust part 38 may be variously modified within such a technical conception.

The case unit 30 in accordance with the embodiment includes a lower case 34 provided with the suction part 36, and an upper case 32 connected to the upper portion of the lower case 34 and provided with the exhaust part 38.

An electronic unit 44 in which electronic parts are installed on a printed circuit board is installed within the upper case 32, and the heat source member 50, such as a hard disk or a central processing unit, is installed under the electronic unit 44.

Since such a heat source member 50 contacts a channel of air introduced through the suction part 36 and moved to the exhaust part 38, the heat source member 50 may be cooled.

In order to improve cooling efficiency of the heat source member 50, a separate heat dissipation unit 60 may be installed on the heat source member 50.

Since the heat dissipation unit 60 cooling the heat source member 50 through thermal conduction is formed by molding using a material exhibiting a high thermal conductivity and has a large area contacting air to achieve heat exchange with the air, the heat source member 50 may be more effectively cooled.

The heat dissipation unit 60 in accordance with the embodiment includes a heat dissipation body 62 contacting the outer surface of the heat source member 50 and heat dissipation fins 64 protruding from the heat dissipation body 62 to increase a contact area with air.

The heat dissipation body 62 is fixed to the outer surface of the heat source member 50 and contacts the channel of air moving from the suction part 36 to the exhaust part 38.

A plurality of heat dissipation fins 64 is provided at the outer surface of the heat dissipation body 62, thus increasing an area contacting air to exchange heat.

The case 30 is provided with guide diaphragms 40 guiding the channel of air moving from the suction part 36 to the exhaust part 38 to improve cooling efficiency.

Such guide diaphragms 40 may have various shapes within a technical conception in which structures guiding the channel of air introduced into the case unit 30 through the suction part 36 and then moved to the exhaust part 38 are installed within the case unit 30.

The guide diaphragms 40 in accordance with the embodiment protrude along both sides of the channel of air.

The guide diaphragms 40 may connect the suction part 36 and the exhaust part 38 in a straight line, and in order to increase the velocity of air using Bernoulli's theorem to improve cooling efficiency of the heat source member 50, the guide diaphragms 40 may be configured such that the channel of air at the inside of the guide diaphragms 40 becomes narrow toward the exhaust part 38.

The guide diaphragms 40 protrude along both sides of the channel of air, and thus guide movement of air.

The guide diaphragms 40 in accordance with the embodiment may be formed integrally with the inner surface of the lower case 34 by molding, or may be manufactured separately and then fixed to the lower caser 34.

In order to gradually narrow the channel of air moving along the guide diaphragms 40 to increase the velocity of air, an interval between the inner surfaces of the diaphragms 40 is gradually narrowed from the suction part 36 to the exhaust part 38.

That is, since the interval between the inner surfaces of the portions of the diaphragms 40 connected to the suction part 36 is wide and the interval between the inner surfaces of the portions of the diaphragms 40 connected to the exhaust part 38 is narrow, the velocity of air moving from the suction part 36 to the exhaust part 38 along a bottom surface 46 of the lower case 34 and the inner surfaces of the guide diaphragms 40 is gradually increased, and thus natural exhaustion of the air is effectively carried out.

The natural exhaustion type ultrasonic diagnostic apparatus 1 in accordance with the embodiment may omit a separate fan to dissipate heat of the inside of the case unit 30 and a motor to drive the fan, and thus reduces noise generated from the inside of the case unit 30 to 20 decibels or less.

That is, since noise generated from the inside of the case unit 30 is only operating noise generated from the hard disk drive and the central processing unit, the operating noise may be reduced and thus power consumption may be reduced.

Hereinafter, an operating state of the natural exhaustion type ultrasonic diagnostic apparatus 1 in accordance with the embodiment of the present invention will be described in detail with reference to the accompanying drawings.

Air introduced into the case unit 30 through the suction part 36 exchanges heat while contacting the heat dissipation fins 64 installed at the outside of the heat source member 50, and thereby, the air is heated and the heat source member 50 is cooled.

The heated air moves upwardly by convection, thus moving to the exhaust part 38 located above the suction part 36.

Further, since the interval between the guide diaphragms 40 guiding the channel of the heated air is gradually narrowed from the suction part 36 to the exhaust part 38, the velocity of the air increases due to Bernoulli's theorem. Therefore, the air moving from the suction part 36 to the exhaust part 38 effectively flows and cooling efficiency of the heat source member 50 is improved.

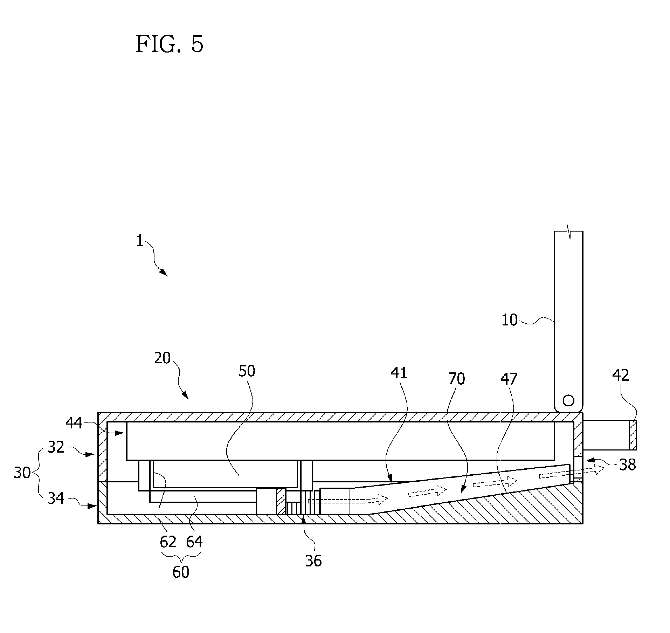

Hereinafter, a natural exhaustion type ultrasonic diagnostic apparatus 1 in accordance with another embodiment of the present invention will be described with reference to FIG. 5.

FIG. 5 is a sectional view schematically illustrating an inclined guide part of the natural exhaustion type ultrasonic diagnosis device in accordance with this embodiment of the present invention.

As shown in FIG. 5, a lower case 34 includes an inclined guide part 70 in which a bottom surface 47 is inclined toward an exhaust part 38. Thereby, a channel of air moving between the inclined guide part 70 and an electronic unit 44 is gradually narrowed, and thus the velocity of the air toward the exhaust part 38 increases.

That is, since the bottom surface 47 of the lower case 34 adjacent to the exhaust part 38 is upwardly inclined toward the exhaust part 38 (based on FIG. 5) and a moving area of air moving along the inner surfaces of guide diaphragms 41 installed along the inclined guide part 70 is narrowed, the velocity of the air increases due to Bernoulli's theorem and thus cooling efficiency of the heat source member 50 is improved.

In the natural exhaustion type ultrasonic diagnostic apparatus having the above-described configuration in accordance with this embodiment, since the exhaust part 38 is located at a position higher than the suction part 36, heated air is naturally exhausted to the outside using convection of air, thereby reducing operating noise and power consumption.

Further, since the channel for the air is gradually narrowed from the suction part 36 to the exhaust part 38, the velocity of the air toward the exhaust part 38 increases due to Bernoulli's theorem, thus improving cooling efficiency of the inside of the case unit 30.

Although a few embodiments of the present invention have been shown and described, it would be appreciated by those skilled in the art that changes may be made in these embodiments without departing from the principles and spirit of the invention, the scope of which is defined in the claims and their equivalents.

That is, although a natural exhaustion type ultrasonic diagnostic apparatus used as a hand-carried ultrasound (HCU) system has been exemplarily illustrated, the natural exhaustion type ultrasonic diagnostic apparatus in accordance with [[one]] an embodiment of the present invention may be applied to other apparatuses requiring dissipation of heat from the insides thereof.

## Claims

1. A natural exhaustion type ultrasonic diagnostic apparatus comprising:
a case unit forming an outer portion of a control panel for ultrasonic diagnostic apparatuses;
a suction part provided at a lower portion of the case unit and forming a path through which air is sucked into the case unit; and
an exhaust part provided at an upper portion of the case unit and forming a path through which the air introduced into the case unit through the suction part is discharged to outside the case unit.

2. The natural exhaustion type ultrasonic diagnostic apparatus according to claim 1, wherein the case unit includes:
a lower case provided with the suction part; and
an upper case connected to an upper portion of the lower case and provided with the exhaust part.

3. The natural exhaustion type ultrasonic diagnostic apparatus according to claim 2, wherein the lower case includes an inclined guide part provided with a bottom surface inclined toward the exhaust part.

4. The natural exhaustion type ultrasonic diagnostic apparatus according to claim 1, wherein the case unit includes guide diaphragms to guide a channel of air introduced into the case unit through the suction part and moved to the exhaust part.

5. The natural exhaustion type ultrasonic diagnostic apparatus according to claim 4, wherein the guide diaphragms protrude along both sides of the channel of air.

6. The natural exhaustion type ultrasonic diagnostic apparatus according to claim 5, wherein the channel of air formed between the guide diaphragms is gradually narrowed toward the exhaust part.

7. The natural exhaustion type ultrasonic diagnostic apparatus according to claim 1, wherein a heat source member contacts a channel of air introduced into the case unit through the suction part and moved to the exhaust part.

8. The natural exhaustion type ultrasonic diagnostic apparatus according to claim 7, further comprising a heat dissipation unit to cool the heat source member by thermal conduction.

9. The natural exhaustion type ultrasonic diagnostic apparatus according to claim 8, wherein the heat dissipation unit includes:
a heat dissipation body contacting an outer surface of the heat source member; and
heat dissipation fins protruding from the heat dissipation body to increase a contact area with air.

10. The natural exhaustion type ultrasonic diagnostic apparatus according to claim 7, wherein noise generated from inside the case unit is 20 decibels or less.
